# EUROPEAN PATENT APPLICATION

(11) **EP 4 379 023 A1**
(43) Date of publication of application: **05.06.2024**
(21) Application number: 22211252.6
(22) Date of filing: 02.12.2022
(51) Int. Cl.: C10G 2/00, B01J 23/75, B01J 27/22, B01J 37/08

(54) **FISCHER-TROPSCH CATALYST ACTIVATION**

(71) Applicant: BP P.L.C., London SW1Y 4PD (GB)
(72) Inventor: The designation of the inventor has not yet been filed
(74) Representative: Hamer, Christopher K.

(57) **Abstract**

A process for converting a mixture of hydrogen and carbon monoxide to a hydrocarbon composition comprising one or more optionally oxygenated hydrocarbons, the process comprising the following steps:
(a) providing a first catalyst material comprising cobalt (e.g., in the form of oxide) disposed on a support;
(b) contacting the first catalyst material with a first reducing agent at a first temperature (T1) and a first pressure (P1) to form a reduced catalyst material;
(c) contacting the reduced catalyst material with carbon monoxide at a second pressure (P2) and a second temperature (T2) to provide a passivated catalyst material, wherein P2 is at least 5 bara, and T2 is at most 250 °C;
(d) contacting the passivated catalyst material with a second reducing agent at a third temperature (T3) and a third pressure (P3) to form an activated catalyst material, P2 is greater than or equal to P3, and wherein T3 is at least 300 °C; and
(e) contacting the activated catalyst material with a mixture of hydrogen and carbon monoxide.

## Description

### BACKGROUND OF THE DISCLOSURE

### Field

The present disclosure relates to a process for activating a Fischer-Tropsch catalyst, a Fischer-Tropsch process using such activated catalysts, and a method of improving at least one aspect of the performance of a Fischer-Tropsch catalyst.

### Technical Background

The conversion of synthesis gas into hydrocarbons by the Fischer-Tropsch process has been known for many years. The growing importance of alternative energy sources has resulted in renewed interest in the Fischer-Tropsch (FT) process as it allows a direct and environmentally acceptable route to high-quality fuels and feedstock chemicals.

FT processes are known for producing linear hydrocarbons for use in fuels, as well as oxygenates which serve as valuable feedstock chemicals. The hydrocarbon fuel deriving from FT processes is better able to meet increasingly stringent environmental regulations compared to conventional refinery-produced fuels, as FT-derived fuels typically have lower contents of sulfur, nitrogen, and aromatic compounds which contribute to the emission of potent pollutants such as SO₂, NOₓ, and particulates. Alcohols derived from FT processes often have a higher-octane rating than hydrocarbons and thus burn more completely, thereby reducing the environmental impact of such a fuel. Alcohols and other oxygenates obtained may also be used as reagents in other processes, such as in the synthesis of lubricants.

A variety of transition metals have been identified to be catalytically active in the conversion of synthesis gas into hydrocarbons and oxygenated derivatives thereof. In particular, cobalt, nickel, ruthenium and iron have been studied, often in combination with a support material, of which the most common are alumina, silica and carbon.

In the typical preparation of supported cobalt-containing FT synthesis catalysts, a solid support material is contacted with a solution of a soluble cobalt compound, such as cobalt nitrate. The impregnated support is subsequently calcined and/or oxidized to form a cobalt oxide, typically one or more of CoO, Co₂O₃, or Co₃O₄. However, such oxides typically have poor FT catalytic activity and must be reduced to form the preferred catalytically active species of cobalt metal. During calcination, cobalt oxides crystallites form on the support material. The properties of these crystallites, being precursors to the active catalyst, are known to have an effect on both the activity and selectivity of the final FT catalyst.

Prior to starting up the Fischer-Tropsch synthesis reaction, the FT catalyst is required to be in the reduced catalyst form of FT catalyst (e.g., cobalt metal). The reduction of the FT catalyst typically occurs in the FT synthesis reactor, although it can occur remotely from the reactor with the catalyst either being reversibly passivated or held an inert conditions to prevent re-oxidation of the catalyst, for example by encasing the catalyst in wax.

The process by which the FT catalyst is activated, is known to have an impact on the performance of the Fischer-Tropsch synthesis reaction and as such is typically performed under conditions which are different to the conditions of the Fischer-Tropsch synthesis reaction.

Accordingly, there exists a need to develop new methods of activating FT catalysts.

### SUMMARY

The inventors have found a process to generate a highly active catalyst composition for use in FT synthesis. In particular, the process, and compositions derived thereof, have been found to generate an FT catalyst which benefit from at least one performance improvement compared to FT catalysts which have not been activated according to the process of the present invention. Additionally, the process also allows for the creation of intermediate catalyst materials that resist environmental oxidation; notably, this can allow the ex-situ preparation of a passivated catalyst intermediate that may be subsequently stored, transported, and introduced into a Fischer-Tropsch reactor, where the activation is completed under mild conditions with only the formation of inert by-products upon the final reduction. As an additional advantage of the ex-situ preparation of a passivated catalyst intermediate followed by completion of the activation in the Fischer-Tropsch reactor is that the final activation conditions are mild and as such the Fischer-Tropsch reactor and gas supply equipment do not need to be able to reach abnormally high pressures and temperatures, lowering equipment costs at the point of use.

Thus, in one aspect, the present disclosure provides a process for converting a mixture of hydrogen and carbon monoxide to a hydrocarbon composition comprising one or more optionally oxygenated hydrocarbons, the process comprising the following steps:
(a) providing a first catalyst material comprising cobalt (e.g., in the form of oxide) disposed on a support;
(b) contacting the first catalyst material with a first reducing agent at a first temperature (T1) and a first pressure (P1) to form a reduced catalyst material;
(c) contacting the reduced catalyst material with carbon monoxide at a second pressure (P2) and a second temperature (T2) to provide a passivated catalyst material, wherein P2 is at least 5 bara, and T2 is at most 250 °C;
(d) contacting the passivated catalyst material with a second reducing agent at a third temperature (T3) and a third pressure (P3) to form an activated catalyst material, P2 is greater than or equal to P3, and wherein T3 is at least 300 °C; and
(e) contacting the activated catalyst material with a mixture of hydrogen and carbon monoxide.

In certain embodiments as otherwise described herein, a passivated catalyst material as otherwise described herein comprises at least 5 wt% (e.g., at least 25%, at least 50%, or even at least 75%) of the cobalt of the passivated catalyst material is in the form of cobalt carbide.

In certain embodiments as otherwise described herein, an activated catalyst material as otherwise described herein comprises hcp cobalt metal in a ratio of at least 50:50 to fcc cobalt metal as measured by x-ray diffraction. In certain desirable embodiments as otherwise described herein, the hcp metal is present in a ratio of at least 75:25 (e.g., at least 80:20 or at least 85:15) to fcc cobalt metal as measured by x-ray diffraction.

In some embodiments, all of steps (a) to (e) of the process are performed in a Fischer-Tropsch reactor.

In some embodiments, steps (a) to (c) of the process are performed ex-situ of the Fischer-Tropsch reactor, and steps (d) and (e) of the process are performed in a Fischer-Tropsch reactor.

In certain embodiments, step (c) is performed in a two-step process as follows:
(c-i) contacting the reduced catalyst material with carbon monoxide at a pressure of at least 1 bara and at most 5 bara to provide a passivated catalyst material; and
(c-ii) contacting the passivated catalyst material with carbon monoxide at the second pressure (P2) of at least 5 bara and a second temperature (T2) of at most 250 °C.

In certain embodiments, steps (a) to (c-i) of the process are performed ex-situ of the Fischer-Tropsch reactor, and steps (c-ii) to (e) of the process are performed in a Fischer-Tropsch reactor.

Another aspect of the present disclosure is the use of a catalyst activation process as described herein to increase the selectivity of the conversion of carbon monoxide and hydrogen to hydrocarbons having five or more carbon atoms (C5+), compared to a catalyst which has been activated using reduction alone.

Another aspect of the present disclosure is the use of a catalyst activation process as described herein to increase the conversion of carbon monoxide and hydrogen to hydrocarbons compared to a catalyst which has been activated using reduction alone.

Another aspect of the present disclosure is the use of a catalyst activation process as described herein to increase the catalyst life of the catalyst.

Other aspects of the disclosure will be apparent to those skilled in the art in view of the description that follows.

### DETAILED DESCRIPTION

The present disclosure is concerned with the Fischer-Tropsch synthesis process and processes to improve the performance of Fischer-Tropsch synthesis processes using cobalt-based catalysts.

The present inventors have found that treatment of a reduced catalyst material with carbon monoxide can form metal carbides, such as cobalt carbides, that result in a passivated catalyst material. The passivated catalyst material can then be converted to an activated catalyst material through treatment with a second reducing agent, and such activated catalyst material can then be used in the conversion of hydrogen and carbon monoxide to hydrocarbon compositions. It has surprisingly been found by the inventors that the pressure at which the carbon monoxide treatment is performed can have a significant impact on the performance of the catalyst in the Fischer-Tropsch synthesis reaction.

Advantageously, the formation of metal carbides, such as cobalt carbides, results in a catalyst passivated material that resists atmospheric oxidation, or other degradation, and so may be stored and/or transported, as metal carbides cannot be further oxidized by molecular oxygen or other common oxidizing agents. Further, metal carbides are known for their exceptional hardness, resulting in a more mechanically robust passivated catalyst material, better able to withstand mechanical stresses which may occur during transportation between sites and handling, e.g., during loading into a reactor. Moreover, the inventors have found that a cobalt carbide-containing passivated catalyst material can be easily converted to an activated catalyst material at relatively mild conditions, yielding methane as the chief byproduct. This production of methane is superior to conventional activation processes, for instance reduction from an oxide using hydrogen, which result in undesirable byproducts such as water. Under these conditions, water can be a potent oxidant, inhibit reduction, promote cobalt sintering and be detrimental to the catalyst and reactor functioning. And, as the conditions for activation according to the present disclosure are mild, high pressure hydrogen equipment may not be necessary for the activation, simplifying plant capital requirements. In fact, in many cases, the carbided material can be converted to activated catalyst under the Fischer-Tropsch reaction conditions themselves.

Such an approach can allow the physical separation of catalyst preparation and Fischer-Tropsch synthesis, with stable passivated catalyst being made at a first location and transported, in some cases over many miles, to a Fischer-Tropsch reactor in which the catalyst is depassivated by activation under relatively mild conditions, then used in a Fischer-Tropsch synthesis. This is especially advantageous, as reactor requirements for the two processes are generally rather different. Further, this approach allows the use of decentralized Fischer-Tropsch reactors that do not need to be capable of conventional, high-temperature, high-pressure catalyst activation. Accordingly, far fewer high-temperature, high-pressure reactors can be used to activate catalyst for many Fischer-Tropsch reactors, which can have lower-temperature and/or lower-pressure capabilities.

Cobalt metal as commonly formed in an activated form of a Fischer-Tropsch catalyst material (e.g., from reduction from cobalt oxide) is typically made up of a mixture of two metallic phases: hexagonal close-packed (hcp) cobalt and face-centered cubic (fcc) cobalt. As the energy difference between these two phases is small, both phases are typically present in substantial amounts. The present inventors have noted that hcp cobalt is more active for FT processes than the typical mixed-phase cobalt. See, e.g., Journal of Catalysis 277, 14-26 (2011). Advantageously, while reduction of cobalt oxide provides a mixture of hcp cobalt and fcc cobalt, reduction of the cobalt carbide-based passivated catalyst surprisingly generates a catalyst that includes cobalt metal almost exclusively in the hcp phase.

Accordingly, one aspect of the disclosure provides a process for converting a mixture of hydrogen and carbon monoxide to a hydrocarbon composition comprising one or more optionally oxygenated hydrocarbons, the process comprising the following steps:
(a) providing a first catalyst material comprising cobalt (e.g., in the form of oxide) disposed on a support;
(b) contacting the first catalyst material with a first reducing agent at a first temperature (T1) and a first pressure (P1) to form a reduced catalyst material;
(c) contacting the reduced catalyst material with carbon monoxide at a second pressure (P2) and a second temperature (T2) to provide a passivated catalyst material, wherein P2 is at least 5 bara, and T2 is at most 250 °C;
(d) contacting the passivated catalyst material with a second reducing agent at a third temperature (T3) and a third pressure (P3) to form an activated catalyst material, P2 is greater than or equal to P3, and wherein T3 is at least 300 °C; and
(e) contacting the activated catalyst material with a mixture of hydrogen and carbon monoxide

The catalyst materials described herein include cobalt, in various forms, a support, and optionally other metals or reaction modifiers. Supported cobalt-based materials are well-known in the art, and can generally be adapted for use in the processes and materials described herein.

In certain embodiments as otherwise described herein, the catalyst materials as described herein include cobalt in the range of 5 wt% to 35 wt%, on an elemental basis. For example, in certain embodiments as otherwise described herein, cobalt may be present in the range of 7-35 wt%, or 10-35 wt%, or 5-25 wt%, or 7-25 wt%, or 10-25 wt%, or 5-20 wt%, or 7-20 wt%, or 10-20 wt%.

The catalyst materials as described herein can include other metal species, e.g., as promoters. For example, in certain embodiments as otherwise described herein, a catalyst material includes manganese, for example, in an amount in a range of up to 15 wt%, e.g., up to 12 wt%, or up to 10 wt%, or up to 7 wt%, on an elemental basis. In certain such embodiments, a catalyst material includes manganese in an amount in the range of 2-15 wt%, e.g., 3-15 wt%, or 4-15 wt%, or 2-12 wt%, or 3-12 wt%, or 4-12 wt%, or 2-10 wt%, or 3-10 wt%, or 4-10 wt%, or 2-7 wt%, or 3-7 wt%, or 4-7 wt%. Of course, in other embodiments substantially no manganese is present (e.g., less than 0.1 wt% or less than 0.5 wt%) manganese is present. Other metals can be present, e.g., as promoters.

Various support materials are known in the art and may be selected based on the precise requirements of the FT reactor or other chemical, mechanical or economic requirements. In certain embodiments as otherwise described herein, the support comprises at least one of titanium oxide, zirconium oxide, cerium oxide, aluminum oxide, silicon oxide and zinc oxide. In particular embodiments, as otherwise described herein, the support comprises exactly one of titanium oxide, zirconium oxide, cerium oxide, aluminum oxide, silicon oxide and zinc oxide. In another particular embodiment, as otherwise described herein, the support comprises titanium oxide. In another particular embodiment, as otherwise described herein, the support is titanium oxide.

The first catalyst material can be prepared using methods conventional in the art. In certain embodiments, the cobalt is introduced onto the support through the introduction of a solution containing a soluble cobalt salt (e.g., cobalt nitrate) to the support and the combination calcined and/or oxidized, rendering insoluble cobalt particles (e.g., as cobalt oxide) on the support. In certain embodiments, the first catalyst material comprises the combination of the calcined metal (e.g., including calcined cobalt) adhered to the support. In particular embodiments, cobalt of the first catalyst material (i.e., as least a portion of the cobalt, up to the entirety of the cobalt, for example, at least 50%, at least 75%, or at least 90%) is in the form of at least one of cobalt oxide and cobalt hydroxide. For example, the cobalt may be cobalt oxide (e.g., CoO, Co₃O₄, or Co₂O₃, or a combination thereof) or cobalt hydroxide (e.g., Co(OH)₂ or Co(OH)₃ or a combination thereof), or a combination of cobalt oxide and cobalt hydroxide.

Following provision of the first catalyst material, the cobalt species disposed thereon, e.g., in the form of cobalt oxide(s)/hydroxide(s) as described above, are substantially reduced to generate a reduced catalyst material. This process results in at least portion of the cobalt being transformed into cobalt metal. Desirably, the reduction results in at least 50 mol% of the cobalt of the reduced catalyst material being in the form of cobalt metal, e.g., at least 75 mol%, or at least 90 mol% of the cobalt being in the form of cobalt metal. For example, in particular embodiments, at least 95 mol% of the cobalt is in the form of cobalt metal). The person of ordinary skill in the art can use conventional methods to reduce cobalt catalyst materials (e.g., cobalt oxide- or cobalt hydroxide-based materials) to metallic form. In certain embodiments as otherwise described herein, the first reducing agent is hydrogen gas, H₂. The hydrogen gas may be mixed with other gases, such as an inert carrier gas. Examples of such inert carrier gasses include nitrogen, carbon dioxide, argon, or helium. The hydrogen gas may also be mixed with carbon monoxide, with or without one or more additional carrier gasses. In certain embodiments, the first reducing gas comprises carbon monoxide, wherein the carbon monoxide is present in an amount in the range of 0.1-10 vol%, e.g., 0.1-5 vol%, or 0.1-1 vol%. But in other embodiments, substantially no carbon monoxide is present (i.e., no more than 0.1 vol%). In certain embodiments, the reduction is effected by contacting the first catalyst material with a first reducing gas, wherein the first reducing gas comprises the first reducing agent, wherein the first reducing gas comprises at least 50 vol% H₂ (e.g., at least 60 vol%, or at least 70 vol%, or at least 80 vol%, or at least 90 vol%, or at least 95 vol%, or essentially 100 vol% H₂).

The reduction of the first catalyst material to provide the reduced catalyst material is performed at a first temperature (T1). In certain embodiments as otherwise described herein, the first temperature is in the range of 200 °C to 500 °C. For example, in certain embodiments, the first temperature is in the range of 250 °C to 400 °C, or in the range of 260 °C to 350 °C, or in the range of 270 °C to 330 °C, or in the range of 280 °C to 320 °C, or in the range of 290 °C to 310 °C. In certain embodiments, the first temperature is approximately 300 °C. The reduction of the first catalyst material to the reduced catalyst material occurs at a first pressure. In certain embodiments as otherwise described herein, the first pressure (P1) is in the range of 0.5 bara to 35 bara, e.g., 0.7 bara to 30 bara. The reduction can be performed for a time (e.g., up to 48 hours, e.g., 2-48 hours or 8-30 hours) and under conditions sufficient to provide the desired degree of reduction as described above.

As described herein, the treatment of the first catalyst material with the first reducing agent produces a reduced catalyst material that includes cobalt as cobalt metal (for example, in an amount of at least 50 mol%, e.g., at least 75 mol%, or at least 90 mol%, or at least 95 mol% of the cobalt as described above). In certain embodiments as otherwise described herein, the cobalt metal of the reduced catalyst material comprises significant amounts of both fcc cobalt metal as well as hcp cobalt metal. In particular embodiments, the cobalt metal includes fcc cobalt metal and hcp cobalt metal present in a ratio in the range of 25:75 to 75:25.

In typical Fischer-Tropsch processes, this reduced catalyst material would be itself be suitable for use as a catalyst. The present inventors have determined, however, that conversion of this reduced catalyst material into carbide form can provide a passivated catalyst material that can be stable for transportation and handling over relatively long distances and times, and also be converted by later reduction to substantially hcp cobalt metal, which the inventors have noted is more active in Fischer-Tropsch processes than the fcc form.

The present inventors have further determined that the pressure at which the reduced catalyst is contacted with carbon monoxide in the conversion of the reduced catalyst material into carbide form can have an effect on the performance of the catalyst when employed in the Fischer-Tropsch process.

The passivated catalyst material is formed by contacting the reduced catalyst material with carbon monoxide. Advantageously, the passivated catalyst material includes cobalt carbide). The relative proportions of metal species may be ascertained by any suitable techniques known to the skilled person, such as fitting x-ray diffractograms. In certain embodiments as otherwise described herein, at least 25 mol%, or at least 50 mol% of the cobalt of the passivated catalyst material is in the form of cobalt carbide as measured by x-ray diffraction. For example, in some embodiments, at least 75 mol% or even at least 90 mol% of the cobalt of the passivated catalyst material is in the form of cobalt carbide. Of course, in other embodiments, passivation can be effective even at lower conversions to carbide, e.g., when surface carbide of a catalyst particle is sufficient to protect cobalt metal interior to the particle.

The reaction of the reduced catalyst material to the passivated catalyst material is performed at a second pressure (P2) and a second temperature (T2), wherein P2 is at least 5 bara and T2 is at most 250 °C. In certain embodiments as otherwise described herein, the P2 is the range of from 5 bara to 50 bara, e.g., 5-30 bara, or 5-25 bara, or 7-50 bara, or 7-30 bara, or 7-25 bara, or 10-50 bara, or 7-30 bara, or 7-25 bara, or 10-50 bara, or 10-30 bara, or 10-25 bara. The carbon monoxide can be provided at a variety of concentrations in a process gas, e.g., at least 5 vol%, e.g., at least 25 vol%, at least 50 vol%, or at least 75 vol%; the process gas can further include, e.g., inert gases such as nitrogen. In certain embodiments, the T2 is in the range of 25 °C to 250 °C, e.g., 50 °C to 225 °C. For example, in particular embodiments, the T2 is in the range of 25 °C to 200 °C, or 75 °C to 200 °C, or 100 °C to 200 °C, or 125 °C to 200 °C. In particular embodiments, T2 is about 200 °C. In other embodiments, the T2 is no more than 150 °C, or no more than 100 °C. For example, in certain embodiments, T2 is in the range of 25 °C to 150 °C, e.g., 50 °C to 125 °C, or 50 °C to 100 °C. The person of ordinary skill in the art will perform the passivation with carbon monoxide under conditions (e.g., temperature, pressure, time) that provides a desired degree of conversion to carbide.

Advantageously, the processes according to the present disclosure results in the formation of a passivated catalyst material as an intermediate product, and that such a passivated catalyst material has improved resistance to atmospheric oxidation. This is affected by chemically protecting at least a portion of the cobalt metal as the carbide. As described elsewhere, the carbide can be then converted to the activated catalyst material. One benefit of this approach is that the passivated catalyst material may be formed in one reactor, which may be specially suited or optimized towards the catalyst passivation process, and then transported to another reactor for use in a FT process with a minimized risk of forming undesirable metal oxides. Given industry trends towards FT reactor decentralization, it may be beneficial in some circumstances to be able to geographically separate catalyst production and catalyst use in FT processes.

Accordingly, in certain embodiments the process as otherwise described herein further comprises packaging the passivated catalyst material. The passivated catalyst material can be packaged in a closed container like a drum, a bag, a box, a canister, or a modular catalyst container. Likewise, in certain embodiments as otherwise described herein, the process further comprises transporting the passivated catalyst material to a location distant from the reactor in which it is passivated. In particular embodiments, the transportation is to a different reactor, or to a different building, a different site, a different facility, or a different plant. In certain such embodiments, the location distant the reactor in which the passivated catalyst material is passivated is at least one mile therefrom, e.g., at least ten miles therefrom, at least twenty miles therefrom, at least fifty miles therefrom, or at least one hundred miles therefrom.

For example, the passivated catalyst material may be produced at a first reactor, optionally packaged, and then transported to a second reactor. In particular embodiments, the first and second reactors are not equivalent (e.g., the first reactor may be optimized for production of the passivated catalyst material, and the second reactor may be optimized for a FT process). Notably, the second reactor need not be built to the same specifications as the first reactor; as FT processes are typically performed under conditions less stringent than those for the initial reduction, lower grades of materials can be used in the FT reactors in which the passivated catalysts are activated and used than those in which the initial reduction is performed.

Advantageously, the passivation techniques and passivated catalysts described herein can allow for reactor specialization, in which a first reactor can be efficiently constructed, scaled and operated to produce the passivated catalyst material, while a second reactor can be efficiently constructed, scaled and operated for the relatively mild activation of the passivated material and subsequent Fischer-Tropsch synthesis. For instance, the emerging field of biomass conversion suitably uses relatively smaller Fischer-Tropsch reactors that can be placed close to the site of biomass generation, such as municipal waste or agricultural products or agricultural waste.

The packaging and transport of the passivated catalyst also allows for lower risk in the catalyst production process as a standardized passivated catalyst can be produced and quality-checked before shipment to the Fischer-Tropsch reactor site. This can allow optimization and standardization of the passivation protocol in ways that may be unavailable to the Fischer-Tropsch reactor. Further, offsite catalyst production allows identification of catalyst batches that do not meet certain specifications and removes reliance on the Fischer-Tropsch reactor and operators thereof to consistently produce highly-active catalyst material. As synthesis reactor downtime can be costly, any process steps that reduce risk of reactor shutdown (e.g., due to an improperly prepared catalyst), or speed catalyst preparation, such as those of the present disclosure, are very valuable.

Accordingly, another aspect of the disclosure is a passivated catalyst material for the Fischer-Tropsch synthesis of a hydrocarbon composition from a gaseous feed comprising carbon monoxide and hydrogen, the passivated catalyst material comprising cobalt disposed on a support, wherein at least 25 mol% (e.g., at least 50 mol%) of the cobalt of the passivated catalyst material is in the form of cobalt carbide as measured by x-ray diffraction. In certain desirable embodiments, at least 75 mol% (e.g., at least 90 mol%) of the cobalt of the passivated catalyst material is in the form of cobalt carbide as measured by x-ray diffraction The amount of cobalt and the amount and identities of any other metals, the support, and other particular characteristics of the passivated catalyst material can be as described above. The passivated catalyst materials can be made as described herein, and can be packaged as described above.

As described above, the passivated catalyst material is then contacted with a second reducing agent at a second temperature and a second pressure to convert at least a portion of the cobalt carbide to cobalt metal and thus form an activated catalyst material which is suitable for use in a FT process

In cases where the passivated catalyst is formed in a reactor which is separate from the reactor used to perform the Fischer-Tropsch reaction, the catalyst has to be loaded in to a reactor zone prior to the treatment using the second reducing agent. Accordingly, when the passivated catalyst material is transported from elsewhere, the method can include unpackaging the passivated catalyst material (e.g., from the closed container, e.g., drum, bag, box, canister, or modular catalyst container in which it is packaged.

Notably, the second reducing agent may be selected to efficiently convert a desired portion, or the entirety, of the cobalt carbide to cobalt metal. In certain embodiments, the amount of cobalt carbide in the activated catalyst material (i.e., as a fraction of total cobalt) is no more than 50 mol% of the amount of cobalt carbide in the passivated catalyst material (i.e., as a fraction of total cobalt). For example, in certain embodiments as otherwise described herein, the amount of cobalt carbide in the activated catalyst material (i.e., as a fraction of total cobalt) is no more than 25 mol%, e.g., no more than 10 mol%, or no more than 5 mol%, or no more than 1 mol% of the amount of cobalt carbide in the passivated catalyst material (i.e., as a fraction of total cobalt). Additionally, it is possible that some cobalt metal remains from the formation of the reduced catalyst material which was not transformed into a carbide, and was further not oxidized during handling of the passivated catalyst material. In certain embodiments as otherwise described herein, in the activated catalyst material at least 50 mol% of the cobalt is in the form of cobalt metal, e.g., at least 75 mol% or at least 90 mol%, or at least 95 mol%, or at least 99 mol% of the cobalt is in the form of cobalt metal.

A surprising effect of the processes and materials described herein is that the cobalt metal formed as part of the activated catalyst material has been unexpectedly found to have a larger proportion of hcp cobalt metal compared to the cobalt metal formed by the conventional reduction of metal oxides (e.g., formed during the production of the reduced catalyst material as otherwise described herein and in conventional reductions of cobalt oxide/hydroxide-based catalyst materials for use in FT processes), and concomitantly less of the other common cobalt metal phase, fcc cobalt metal. This is advantageous as hcp cobalt metal is found to exhibit a higher activity for FT processes compared to fcc cobalt metal. The higher activity of hcp cobalt metal means that less catalyst can be used to achieve the same throughput as conventional catalysts, and/or the reactor may be operated at lower temperatures. Both of these effects can result in significant cost savings. Accordingly, in certain embodiments as otherwise described herein, the activated catalyst material comprises cobalt metal, wherein the cobalt metal comprises hcp cobalt metal in a ratio of at least 50:50 (e.g., at least 60:40 or at least 70:30) to fcc cobalt metal, as measured by x-ray diffraction. For example, in certain embodiments as otherwise described herein, the cobalt metal comprises hcp cobalt metal in a ratio of at least 75:25 (e.g., at least 80:20, or at least 85:15) to fcc cobalt metal. This is contrasted with lower ratios (typically 50:50 or less) in conventional materials. Amounts of hcp and fcc cobalt can be determined using x-ray diffractometry.

In certain embodiments, the second reducing agent is the same as the first reducing agent. In other embodiments, the second reducing agent is different from the first reducing agent. In particular embodiments, the second reducing agent is hydrogen gas, H₂. The hydrogen gas may be mixed with other gases, such as an inert carrier gas. Examples of such inert carrier gasses include nitrogen, carbon dioxide, argon, or helium. The hydrogen gas may also be mixed with carbon monoxide, with or without one or more additional carrier gasses. In certain embodiments, the reduction is effected by contacting the passivated catalyst material with a second reducing gas, wherein the second reducing gas comprises the second reducing agent, wherein the second reducing gas comprises at least 50 vol% H₂ (e.g., at least 60 vol%, or at least 70 vol%, or at least 80 vol%, or at least 90 vol%, or at least 95 vol%, or essentially 100 vol% H₂).

The reduction of the passivated catalyst material to the activated catalyst material is performed at a third temperature (T3) and a third pressure (P3), wherein P3 is greater than or equal to P2 and T3 is at least 300 °C. In certain embodiments as otherwise described herein, T3 is in the range of 300 °C to 500 °C. For example, in certain embodiments, T3 is in the range of 300-490 °C, or 300-480 °C, or 300-470 °C, or 300-460 °C, or 300-450 °C. In certain embodiments as otherwise described herein, P3 is in the range of 1 to 50 bara, for example in the range of from 2 bara to 35 bara. Examples of suitable pressure ranges for P3 include 2-50 bara, or 2-35 bara, or 2-30 bara, or 5-30 bara, or 5-25 bara, or 7-50 bara, or 7-30 bara, or7-25 bara, or 10-50 bara, or 7-30 bara, or 7-25 bara, or 10-50 bara, or 10-30 bara, or 10-25 bara. The reduction can be performed for a time and under conditions sufficient to provide the desired degree of reduction as described above. For example, the reduction of the passivated catalyst material to the activated catalyst material can be performed for a time in the range of 2 hours to 48 hours, or 2 hours to 36 hours, or 2 hours to 24 hours, or 2 hours to 12 hours, or 2 hours to 8 hours. For example, the reduction may be performed for a time in the range of 3 hours to 48 hours, or 4 hours to 48 hours, or 5 hours to 48 hours, or 5 hours to 36 hours, or 5 hours to 24 hours.

In certain embodiments, the conditions of a Fischer-Tropsch process are suitable for the activation of the passivated catalyst material, and so separate conditions are not required.

Once generated, the activated catalyst material is suitable for use in an FT process. Accordingly, the activated catalyst material is contacted with a with a gaseous mixture comprising carbon monoxide and hydrogen, to form the hydrocarbon composition.

In some embodiments, all of steps (a) to (e) of the process are performed in a Fischer-Tropsch reactor.

In some embodiments, steps (a) to (c) of the process are performed ex-situ of the Fischer-Tropsch reactor, and steps (d) and (e) of the process are performed in a Fischer-Tropsch reactor.

In certain embodiments, step (c) is performed in a two-step process as follows:
(c-i) contacting the reduced catalyst material with carbon monoxide at a pressure of at least 1 bara and at most 5 bara to provide a passivated catalyst material; and
(c-ii) contacting the passivated catalyst material with carbon monoxide at a second pressure (P2) of at least 5 bara, and a second temperature (T2) of at most 250 °C.

In certain embodiments, steps (a) to (c-i) of the process are performed ex-situ of the Fischer-Tropsch reactor, and steps (c-ii) to (e) of the process are performed in a Fischer-Tropsch reactor.

The person of ordinary skill in the art can adapt conventional FT processes for use of the catalyst materials described herein In certain embodiments of the Fischer-Tropsch processes of the disclosure, the volume ratio of hydrogen to carbon monoxide (H₂:CO) in the gaseous reactant mixture is typically at least 1 : 1, preferably at least 1.1 : 1, more preferably at least 1.2 : 1, more preferably at least 1.3 : 1, more preferably at least 1.4 : 1, more preferably at least 1.5 : 1, or even at least 1.6 : 1. In some or all embodiments of the present invention, the volume ratio of hydrogen to carbon monoxide (H₂:CO) in the gaseous reactant mixture is at most 5 : 1, preferably at most 3 : 1, most preferably at most 2.2 : 1. Examples of suitable volume ratios of hydrogen to carbon monoxide (H₂:CO) in the gaseous reactant mixture include the ranges: from 1 : 1 to 5 : 1; from 1.1 : 1 to 3 : 1 ; from 1.2 : 1 to 3 : 1; from 1.3 : 1 to 2.2 : 1 ; from 1.4 : 1 to 5 : 1 ; from 1.4 : 1 to 3 : 1 ; from 1.4 : 1 to 2.2 : 1 ; from 1.5 : 1 to 3 : 1 ; from 1.5 : 1 to 2.2 : 1 ; and, from 1.6:1 to 2.2:1. The gaseous reactant stream may also comprise other gaseous components, such as nitrogen, carbon dioxide, water, methane and other saturated and/or unsaturated light hydrocarbons, each preferably being present at a concentration of less than 30% by volume.

Conventional Fischer-Tropsch temperatures may be used in order to prepare optionally oxygenated hydrocarbons in accordance with the present disclosure. For example, the temperature of the reaction may suitably be in the range from 100 to 400 °C, such as from 150 to 350 °C, or from 150 to 250 °C. The pressure of the reaction may suitably be in the range from 10 to 100 bar (from 1 to 10 MPa), such as from 15 to 75 bar (from 1.5 to 7.5 MPa), or from 20 to 50 bar (from 2.0 to 5.0 MPa).

In preferred embodiments, the temperature of the Fischer-Tropsch reaction is in the range from 150 to 350 °C, more preferably from 180 to 300 °C, and most preferably from 200 to 260 °C. In preferred embodiments, the pressure of the Fischer-Tropsch reaction is in the range from 10 to 100 bar (from 1 to 10 MPa), more preferably from 10 to 60 bar (from 1 to 6 MPa) and most preferably from 20 to 45 bar (from 2 to 4.5 MPa).

The Fischer-Tropsch synthesis reaction may be performed in any suitable type of reactor, for example it may be performed in a fixed bed reactor, a slurry bed reactor, or a CANS reactor. Specifically with respect to CANS reactors, a passivated catalyst material as described herein can be packaged in a modular catalyst container suitable for use in a reactor tube of the CANS reactor (e.g., offsite then transported to the reactor site). The CANS can be loaded with catalyst and put in the plant for the re-reduction. The CANS design means handling, transporting and storing the catalyst (in CANS) is much easier and this becomes very useful for the process. The catalyst can be loaded in the CAN and reduced and passivated ahead of storage, shipping, and subsequent loading into a Fischer-Tropsch plant where the catalyst may be reactivated. CANS reactors, and associated containers suitable for use in the processes described herein, are described in WO 2011/048361, which is hereby incorporated herein by reference in its entirety for its disclosure of such canisters and uses thereof.

It may be desired that the passivated catalyst material is introduced into the reactor zone before the formation of the activated catalyst material, i.e., such that the activation of the catalyst occurs in situ in the reactor zone. This approach allows loading of the passivated catalyst material, from a first reactor or from another site (e.g., by unpackaging it from a container, or by coupling a canister into the reactor zone). Subsequently, the second reactor may be purged of undesirable components, such as atmospheric oxygen or moisture, prior to formation of the activated catalyst material, allowing the provision of highly active catalyst under carefully controlled conditions. Accordingly, in certain embodiments as otherwise described herein, the process further comprises introducing the passivated catalyst material into the reactor zone.

In certain situations, the passivated catalyst material may replace a previous catalyst material. For example, the previous catalyst material may be a spent catalyst material that has lost activity or selectivity through incidental degradation. As another example, the spent catalyst material may be one optimized for a particular process or product distribution, and a different catalyst is desired which has different optimization. As such, in certain embodiments as otherwise described herein, the method further comprises, before introducing the passivated catalyst material into the reactor zone, removing a spent catalyst material from the reactor zone.

The hydrocarbon composition can vary based on changes in process conditions as known in the art. In certain embodiments, the hydrocarbon composition comprises hydrocarbons (e.g., linear hydrocarbons, branched hydrocarbons, saturated or unsaturated hydrocarbons) and oxygenated derivatives thereof. Examples of the oxygenated derivatives thereof include hydrocarbons with one or more functional group of alcohols, aldehydes, ketones, carboxylic acids, esters, and combinations thereof. In certain embodiments as otherwise described herein, the hydrocarbon composition comprises at least one of alkanes, alkenes, and alcohols.

Another aspect of the present disclosure is the use of a catalyst activation process as described herein to increase the selectivity of the conversion of carbon monoxide and hydrogen to hydrocarbons having five or more carbon atoms (C5+), compared to a catalyst which has been activated using reduction alone.

Another aspect of the present disclosure is the use of a catalyst activation process as described herein to increase the conversion of carbon monoxide and hydrogen to hydrocarbons compared to a catalyst which has been activated using reduction alone.

Another aspect of the present disclosure is the where the catalyst material comprising cobalt (e.g., in the form of oxide) disposed on a support is at least partially oxidized. Wherein at least 10% of the cobalt is in an oxidized form, e.g., at least 20% of cobalt is in an oxidized form, or at least 30% is in an oxidized form, or at least 50% is in an oxidized form. This could be in the form of fully oxidized cobalt or a passivated material with an oxide layer and metallic core.

Since the use of a catalyst activation process as described herein increases the productivity of the catalyst in Fischer-Tropsch reactions, this enables a the person of ordinary skill in the art to decrease the reaction temperature to achieve the same conversion of carbon monoxide and hydrogen to hydrocarbons compared to a catalyst which has been activated using reduction alone, and hence another aspect of the present disclosure is the use of a catalyst activation process as described herein to increase the catalyst life of the catalyst.

Another aspect of the present disclosure provides for a process for converting a mixture of hydrogen and carbon monoxide to a hydrocarbon composition comprising one or more optionally oxygenated hydrocarbons, the process comprising the following steps:
(x) contacting a catalyst material comprising cobalt disposed on a support, wherein the cobalt is present on the support in both metallic and oxidized form, with carbon monoxide at a pressure (Px) and a temperature (Tx) to provide a passivated catalyst material, wherein Px is at least 5 bara, and Tx is at most 250 °C;
(y) contacting the passivated catalyst material prepared in step (x) with a reducing agent at a temperature (Ty) and a pressure (Py) to form an activated catalyst material, Px is greater than or equal to Py, and wherein Ty is at least 300 °C; and
(z) contacting the activated catalyst material with a mixture of hydrogen and carbon monoxide.

In the above process, the pressure Px preferably is a pressure equivalent to P2 and the same preferences for pressure P2 shall apply equally to Px.

In the above process, the temperature Tx preferably is a temperature equivalent to T2 and the same preferences for temperature T2 shall apply equally to Tx.

In the above process, the pressure Py preferably is a pressure equivalent to P3 and the same preferences for pressure P3 shall apply equally to Py.

In the above process, the temperature Ty preferably is a temperature equivalent to T3 and the same preferences for temperature T3 shall apply equally to Ty.

### EXAMPLES

The Examples that follow are illustrative of specific embodiments of the methods of the disclosure, and various uses thereof. They are set forth for explanatory purposes only, and are not to be taken as limiting the scope of the disclosure.

The Fischer-Tropsch catalyst was prepared by impregnation of a titania support with cobalt nitrate hexahydrate, manganese acetate tetrahydrate, followed by drying and calcination at 300 °C. The catalysts contained, after reduction, cobalt in an amount of 10% by weight and manganese in amounts between 1 and 10% by weight.

The catalysts were activated in accordance with the appropriate listed H₂ and CO conditions as detailed below. A Fischer-Tropsch reaction was performed through contacting the respective catalyst with a 1.8 H₂:CO in N₂ at 30 barg and 4300 hr-1 syngas gas hourly space velocity. Applied temperatures of the FT reaction are given in brackets next to the CO conversions. The tests were completed on a high throughput multichannel reactor with 1 g of catalyst using common gas feeds and pressure, with varying applied temperatures. The catalysts were initially dried in nitrogen at 120°C before completing the activation sequence.

**Table 1. Catalyst 10%Co 1%Mn on TiO₂**

| | **Activation Treatment Conditions** | **% CO Conv (applied T)** | **% Selectivity** | | |
|---|---|---|---|---|---|
| | | | **CH₄** | **C2-C4** | **C5+** |
| A (comparative) | H₂ Reduction (200 °C) | 34 (215 °C) | 5.5 | 5.4 | 89 |
| B (comparative) | H₂ Reduction (300 °C) | 36 (215 °C) | 6 | 4.9 | 89 |
| C (comparative) | H₂ Reduction (450 °C) | 27 (215 °C) | 7 | 7.1 | 86 |
| 1 | a) H₂ reduction (300 °C) | 57 (215 °C) | 5.2 | 3.3 | 91.3 |
| | b) CO treatment (6 bara, 230 °C) | | | | |
| | c) H₂ reduction (350 °C) | | | | |

**Table 2. Catalyst 10%Co 10%Mn TiO₂**

| | **Activation Treatment Conditions** | **% CO Conv (applied T)** | **% Selectivity** | | |
|---|---|---|---|---|---|
| | | | **CH₄** | **C2-C4** | **C5+** |
| D (comparative) | H₂ Reduction (300 °C) | 21 (220 °C) | 9.5 | 35 | 55.5 |
| E (comparative) | H₂ Reduction (450 °C) | 27 (220 °C) | 6 | 5.9 | 88.1 |
| 2 | a) H₂ reduction (300 °C) | 47 (215 °C) | 4.9 | 3.4 | 92 |
| | b) CO treatment (6 bara, 230 °C) | | | | |
| | c) H₂ reduction (450 °C) | | | | |

**Table 3. Catalyst 10%Co 10%Mn on TiO₂**

| | **Activation Treatment Conditions** | **% CO Conv (applied T)** | **% Selectivity** | | |
|---|---|---|---|---|---|
| | | | **CH₄** | **C2-C4** | **C5+** |
| F (comparative) | H₂ Reduction (300 °C) | 36 (215 °C) | 6 | 4.9 | 89 |
| G (comparative) | a) H₂ reduction (300 °C) | 38 (215 °C) | 5.1 | 4.7 | 90 |
| | b) CO treatment (1 bara, 230 °C) | | | | |
| | c) H₂ reduction (300 °C) | | | | |
| 3 | a) H₂ reduction (300 °C) | 50 (215 °C) | 4.5 | 3.2 | 92 |
| | b) CO treatment (6 bara, 230 °C) | | | | |
| | c) H₂ reduction (300 °C) | | | | |

**Table 4. Catalyst 10%Co1%MnTiO₂**

| | **Activation** | **Temp (°C)** | **%Conv** | **Selectivity** | |
|---|---|---|---|---|---|
| | | | | **CH4** | **C5+** |
| H (Comp) | 250°C, 16b, 50%H₂ | 205 | 38.3 | 4.9 | 90.8 |
| I (Comp) | a) 200°C, 50%H₂, 4300hr-1, 16b | 205 | 32.8 | 5.2 | 90.2 |
| | b) 230°C 50%CO, 2000hr-1, 10b | | | | |
| | c) 250°C, 50%H₂, 4300hr-1, 1b | | | | |
| 4 | a) 200°C, 50%H₂, 4300hr-1, 16b | 205 | 43.5 | 3.9 | 92.8 |
| | b) 230°C 50%CO, 2000hr-1, 10b | | | | |
| | c) 300°C, 50%H₂, 4300hr-1, 1b | | | | |
| 5 | a) 200°C, 50%H₂, 4300hr-1, 16b | 205 | 45.9 | 4.5 | 92 |
| | b) 230°C 50%CO, 2000hr-1, 10b | | | | |
| | c) 350°C, 50%H₂, 4300hr-1, 1b | | | | |
| 6 | a) 200°C, 50%H₂, 4300hr-1, 16b | 205 | 49.7 | 4 | 93 |
| | b) 230°C 50%CO, 2000hr-1, 10b | | | | |
| | c) 400°C, 50%H₂, 4300hr-1, 1b | | | | |

**Table 5 10%Co 1%Mn TiO₂**

| | **Activation** | **Temp (°C)** | **%Conv** | **Selectivity** | | |
|---|---|---|---|---|---|---|
| | | | | **CH4** | **C5+** | **LPG** |
| J (Comp) | 100%H2,300°C | 205 | 38.3 | 4.9 | 90.8 | 4.2 |
| K (Comp) | a) 300°C, 100%H₂, 4300hr-1, 0b, | 205 | 36.2 | 5.5 | 90.1 | 4.3 |
| | b) 230°C 50%CO, 2000hr-1, 10b, | | | | | |
| | 240°C, 100%H₂, 4300hr-1, 6b, | | | | | |
| 7 | a) 300°C, 100%H₂, 4300hr-1, 0b, | 205 | 52.2 | 4.9 | 91.9 | 2.9 |
| | b) 230°C 50%CO, 2000hr-1, 10b, 300°C, 100%H₂, 4300hr-1, 6b, | | | | | |

As can be seen from the results presented in Table 1 to 5, the examples which comprised a treatment with CO, and then reduction with hydrogen produced a significantly improved performance in conversion of CO at a given applied temperature compared to the example which employed just a standard hydrogen reduction.

The particulars shown herein are by way of example and for purposes of illustrative discussion of certain embodiments of the present disclosure only and are presented in the cause of providing what is believed to be the most useful and readily understood description of the principles and conceptual aspects of various embodiments of the disclosure. In this regard, no attempt is made to show details associated with the methods of the disclosure in more detail than is necessary for the fundamental understanding of the methods described herein, the description taken with the examples making apparent to those skilled in the art how the several forms of the methods of the disclosure may be embodied in practice. Thus, before the disclosed processes and devices are described, it is to be understood that the aspects described herein are not limited to specific embodiments, apparatus, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the methods of the disclosure (especially in the context of the following embodiments and claims) are to be construed to cover both the singular and the plural, unless otherwise indicated herein or clearly contradicted by context.

All methods described herein can be performed in any suitable order of steps unless otherwise indicated herein or otherwise clearly contradicted by context. The use of any and all examples, or exemplary language (e.g., "such as") provided herein is intended merely to better illuminate the methods of the disclosure and does not pose a limitation on the scope of the disclosure. No language in the specification should be construed as indicating any non-claimed element essential to the practice of the methods of the disclosure.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component. As used herein, the transition term "comprise" or "comprises" means includes, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the embodiment to the specified elements, steps, ingredients or components and to those that do not materially affect the embodiment.

All percentages, ratios and proportions herein are by weight, unless otherwise specified.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

Groupings of alternative elements or embodiments of the disclosure are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Some embodiments of various aspects of the disclosure are described herein, including the best mode known to the inventors for carrying out the methods described herein. Of course, variations on these described embodiments will become apparent to those of ordinary skill in the art upon reading the foregoing description. The skilled artisan will employ such variations as appropriate, and as such the methods of the disclosure can be practiced otherwise than specifically described herein. Accordingly, the scope of the disclosure includes all modifications and equivalents of the subject matter recited in the claims appended hereto as permitted by applicable law. Moreover, any combination of the above-described elements in all possible variations thereof is encompassed by the disclosure unless otherwise indicated herein or otherwise clearly contradicted by context.

The phrase "at least a portion" as used herein is used to signify that, at least, a fractional amount is required, up to the entire possible amount.

In closing, it is to be understood that the various embodiments herein are illustrative of the methods of the disclosures. Other modifications that may be employed are within the scope of the disclosure. Thus, by way of example, but not of limitation, alternative configurations of the methods may be utilized in accordance with the teachings herein. Accordingly, the methods of the present disclosure are not limited to that precisely as shown and described.

## Claims

1. A process for converting a mixture of hydrogen and carbon monoxide to a hydrocarbon composition comprising one or more optionally oxygenated hydrocarbons, the process comprising the following steps:
(a) providing a first catalyst material comprising cobalt (e.g., in the form of oxide) disposed on a support;
(b) contacting the first catalyst material with a first reducing agent at a first temperature (T1) and a first pressure (P1) to form a reduced catalyst material;
(c) contacting the reduced catalyst material with carbon monoxide at a second pressure (P2) and a second temperature (T2) to provide a passivated catalyst material, wherein P2 is at least 5 bara, and T2 is at most 250 °C;
(d) contacting the passivated catalyst material with a second reducing agent at a third temperature (T3) and a third pressure (P3) to form an activated catalyst material, P2 is greater than or equal to P3, and wherein T3 is at least 300 °C; and
(e) contacting the activated catalyst material with a mixture of hydrogen and carbon monoxide.

2. The process of claim 1, wherein each catalyst material comprises cobalt in the range of 5 wt% to 35 wt%, on an elemental basis.

3. The process of claim 1 or claim 2, wherein each catalyst material further comprises manganese, ruthenium or rhenium, e.g., in the range of up to 15 wt% on an elemental basis.

4. The process of any one of claims 1 to 3, wherein the first reducing agent is hydrogen, and the first temperature (T1) is in the range of 250-350 °C and the first pressure (P1) is in the range of 0.7 bara to 8 bara.

5. The process of any one of claims 1 to 4, wherein the reduced catalyst material comprises cobalt metal, wherein the cobalt metal comprises hcp cobalt metal and fcc cobalt metal in a ratio in the range of 25:75 to 75:25.

6. The process of any one of claims 1 to 5, wherein at least 20 mol% of the cobalt of the passivated catalyst material is in the form of cobalt carbide as measured by x-ray diffraction.

7. The process of any one of claims 1 to 6, wherein the reduced catalyst material is contacted with carbon monoxide at a second pressure (P2) in the range of from 5 bara to 50 bara.

8. The process of any one of claims 1 to 7, wherein the reduced catalyst material is contacted with carbon monoxide and the second temperature (T2) is in the range of from 25 °C to 250 °C.

9. The process of any one of claims 1 to 8, wherein the contacting with the second reducing agent is a contacting with hydrogen and the third temperature (T3) is in the range of 300 °C to 450 °C.

10. The process of any one of claims 1 to 9, where all of steps (a) to (e) of the process are performed in a Fischer-Tropsch reactor.

11. The process of any one of claims 1 to 9, where steps (a) to (c) are performed ex-situ of the Fischer-Tropsch reactor, and steps (d) and (e) are performed in a Fischer-Tropsch reactor.

12. The process of claim 11, further comprising transporting the passivated catalyst material produced in step (c) to a location distant from the reactor in which it is passivated (e.g., transporting to a different reactor, building, site, or facility).

13. The process of claim 12, wherein the location distant the reactor in which the passivated catalyst material is passivated is at least ten miles therefrom.

14. The process of any one of claims 1 to 9, where step (c) is performed in a two-step process as follows:
(c-i) contacting the reduced catalyst material with carbon monoxide at a pressure of at least 1 bara and at most 5 bara to provide a passivated catalyst material; and
(c-ii) contacting the passivated catalyst material with carbon monoxide at the second pressure (P2) of at least 5 bara.

15. The process of claim 14, where steps (a) to (c-i) are performed ex-situ of the Fischer-Tropsch reactor, and steps (c-ii) to (e) are performed in a Fischer-Tropsch reactor.

16. The process of claim 14 or claim 15, further comprising transporting the passivated catalyst material produced in step (c-i) to a location distant from the reactor in which it is passivated (e.g., transporting to a different reactor, building, site, or facility).

17. The process of claim 16, wherein the location distant the reactor in which the passivated catalyst material is passivated is at least ten miles therefrom.
